# EUROPÄISCHE PATENTSCHRIFT

(11) **EP 2 755 584 B1**
(45) Veröffentlichungstag und Bekanntmachung des Hinweises auf die Patenterteilung: **15.08.2018**
(21) Anmeldenummer: 12751059.2
(22) Anmeldetag: 22.08.2012
(51) Int. Cl.: A61B 17/80, A61B 17/86

(54) **KNOCHENPLATTE**
BONE PLATE
PLAQUE DE CUISSON

(30) Priorität: 12.09.2011 EP 11180964
(43) Veröffentlichungstag der Anmeldung: 23.07.2014
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll
(86) Internationale Anmeldenummer: PCT/EP2012/066353
(87) Internationale Veröffentlichungsnummer: WO 2013/037613

(56) Entgegenhaltungen:
- EP-A1- 1 649 819
- WO-A2-2010/076977
- DE-A1- 19 629 011
- DE-A1-102004 035 546
- DE-A1-102006 062 164
- US-A1- 2008 300 637
- US-A1- 2011 015 681

## Beschreibung

Die Erfindung betrifft eine Knochenplatte mit einem Durchgangsloch, das sich von einer Oberseite bis zu einer knochenseitigen Unterseite der Knochenplatte erstreckt. In dem Durchgangsloch ist eine Lippe ausgebildet, die von der Mantelfläche des Durchgangslochs vorspringt und die sich in Umfangsrichtung des Durchgangslochs erstreckt.

Solche Knochenplatten sind dazu ausgelegt, mit einem Knochen verbunden zu werden. Sie können beispielsweise dazu dienen, einen Knochen nach einem Bruch zu stabilisieren. Dazu wird die Knochenplatte so positioniert, dass sie sich über die Bruchstelle hinweg erstreckt, und dann an den Knochenfragmenten befestigt. Die Bruchstelle wird dadurch ruhig gestellt und der Knochen kann ausheilen. Die Knochenplatte kann auch anderen Zwecken dienen und beispielsweise ein mit einem Knochen zu verbindendes Element einer Endoprothese sein.

Zur Befestigung der Knochenplatte an dem Knochen wird eine Knochenschraube in das Durchgangsloch eingesetzt, die sowohl am Schaft als auch am Kopf mit einem Gewinde versehen ist. Der Schaft der Schraube dringt so weit in das Knochenmaterial ein, dass der Kopf der Schraube in das Durchgangsloch der Knochenplatte gelangt. Der Kopf der Knochenschraube ist so bemessen, dass sein äußerer Durchmesser größer ist als der kleinste Durchmesser des Plattenloches zum Beispiel auf der Höhe der Materiallippe. Beim weiteren Eindrehen der Knochenschraube wird die Lippe verformt, so dass sich eine Gewindeverbindung zwischen dem Kopf der Knochenschraube und der Lippe im Durchgangsloch der Knochenschraube bildet. Indem sowohl zwischen dem Schaft der Knochenschraube und dem Knochenmaterial als auch zwischen dem Kopf der Schraube und der Knochenplatte eine Gewindeverbindung besteht, wird eine sichere Verbindung zwischen der Knochenplatte und dem Knochen hergestellt. Da die Gewindeverbindung zwischen dem Kopf der Knochenschraube und der Knochenplatte erst beim Eindrehen der Knochenschraube durch einen Umformvorgang gebildet wird, ist es nicht erforderlich, die Knochenschraube in einem bestimmten vorgegebenen Winkel in die Knochenplatte einzudrehen. Vielmehr kann der Winkel innerhalb bestimmter Grenzen frei gewählt werden. Dies gibt den Chirurgen eine hohe Flexibilität während der Operation.

Bei bisherigen Knochenplatten erstreckt sich die Lippe über den gesamten Umfang des Durchgangslochs. Um eine stabile Gewindeverbindung herzustellen, wird die Schraube so weit in die Lippe eingeschraubt, bis über den gesamten Umfang des Schraubenkopfs eine Gewindeverbindung mit der Lippe besteht. Um dies zu erreichen, muss die Schraube nach dem ersten Eingriff mit der Lippe noch ein beträchtliches Stück weiter gedreht werden, wobei das Material der Lippe in erheblichem Umfang umgeformt wird.

Aus der US 2008/0300637 A1 ist eine Knochenplatte mit einem Durchgangsloch bekannt, von dessen Umfangsfläche mehrere Lamellen vorspringen, welche dazu ausgebildet sind, in die Gewindeausnehmungen eines Kopfgewindes einer Knochenschraube einzugreifen. Beim Einschrauben der Knochenschraube können die Lamellen leicht nach oben oder unten verbogen werden, um die Ausrichtung der Knochenschraube anzupassen.

Bei jeder Materialumformung besteht das Risiko, dass kleine Partikel von der Oberfläche des Materials abgetragen werden können. Wenn diese Partikel sich im Umfeld der Knochenplatte verteilen und in Kontakt mit dem Gewebe kommen, kann es zu Komplikationen kommen, die den Fortschritt des Heilungsprozesses behindern.

Der Erfindung liegt die Aufgabe zu Grunde, eine Knochenplatte vorzustellen, bei der das Risiko von Komplikationen vermindert ist. Ausgehend vom eingangs genannten Stand der Technik wird die Aufgabe gelöst mit den Merkmalen des unabhängigen Anspruchs. Vorteilhafte Ausführungsformen finden sich in den Unteransprüchen.

Erfindungsgemäß erstreckt sich die Lippe über einen Teil des Umfangs des Durchgangslochs. Ein der Lippe gegenüberliegender Umfangsabschnitt der Mantelfläche ist frei von einer Lippe, wobei der Umfangsabschnitt sich über mindestens 120° erstreckt.

Zunächst werden einige Begriffe erläutert. Eine Lippe bezeichnet einen Bereich, in dem die Materialstärke verglichen mit der Materialstärke der Knochenplatte vermindert ist. Die Lippe springt von der Mantelfläche des Durchgangslochs in Richtung des Zentrums des Durchgangslochs vor. Die Lippe hat eine Längserstreckung entlang dem Umfang des Durchgangslochs.

Wenn die Mantelfläche in einem der Lippe gegenüberliegenden Abschnitt frei von einer Lippe ist, bedeutet dies, dass ein Schraubenkopf, der in das Durchgangsloch eingesetzt wird und der auf der einen Seite in Kontakt mit der Lippe steht, auf der anderen Seite direkten Kontakt mit der Mantelfläche des Durchgangslochs hat.

Indem die Lippe sich nur über einen Teil des Umfangs des Durchgangslochs erstreckt, findet nur in diesem Bereich eine Materialumformung statt. Je weiter der Schraubenkopf in die Lippe eingreift, desto stärker wird die Kraft, mit der der Schraubenkopf gegen die gegenüberliegende Mantelfläche gedrückt wird. Indem der Schraubenkopf einerseits eine Gewindeverbindung mit der Lippe eingeht und andererseits gegen die Mantelfläche gedrückt wird, ist der Schraubenkopf sicher in dem Durchgangsloch fixiert. Verglichen mit einer Lippe, die sich über den gesamten Umfang des Durchgangslochs erstreckt, wird jedoch nur in wesentlich geringerem Ausmaß Material umgeformt. In gleichem Maße sinkt das Risiko, dass es zu Komplikationen durch abgetragene Partikel kommt.

Der Schraubenkopf soll auf der anderen Seite des Durchgangslochs möglichst flächig auf der Mantelfläche aufliegen, damit er sicheren Halt findet. Dies setzt voraus, dass ein hinreichend großer Abschnitt der Mantelfläche frei von einer Lippe ist. Vorzugsweise erstreckt sich der zusammenhängende Umfangsabschnitt, in dem die Mantelfläche frei von einer Lippe ist über mindestens 180°, weiter vorzugsweise über mindestens 240°. Der verbleibende Umfang des Durchgangslochs kann eine durchgehende Lippe aufweisen. Möglich ist auch eine aus mehreren Abschnitten zusammengesetzte Lippe.

In einer bevorzugten Ausführungsform weist die Lippe eine zwischen der Mantelfläche des Durchgangslochs und einem zentralen Bereich der Lippe angeordnete Stufe auf, die sich längs der Lippe erstreckt. Die Lippe weist dann im zentralen Bereich einen Abschnitt auf, in dem die Materialstärke gering ist. Die Knochenschraube, die in diesen Abschnitt der Lippe eingreift, muss zunächst nur wenig Material verdrängen. Trotzdem besteht unmittelbar nach dem ersten Eingriff eine sichere Führung, so dass die Knochenschraube sich beim weiteren Eindrehen entlang einer definierten Bahn bewegt. Erst wenn die Knochenschraube in den Bereich der Stufe vordringt, muss mehr Material verdrängt werden. Die Gewindeverbindung zwischen dem Kopf der Knochenschraube und der Lippe wird damit in zwei Schritten gebildet. In einem ersten Schritt greift das Kopfgewinde in einen Bereich geringer Materialstärke ein, so dass der erste Eingriff leicht und mit geringem Kraftaufwand stattfindet. Erst wenn das Kopfgewinde bis in den Bereich der Stufe vorgedrungen ist, wird so viel Material verdrängt, dass die Gewindeverbindung die hinreichende Stabilität erhält.

Wenn die Lippe mit einer Stufe versehen ist, bedeutet dies, dass, wenn man sich ausgehend von Zentrum in radialer Richtung nach außen bewegt, die Lippe im Bereich der Stufe eine größere Steigung hat als im Bereich des zentralen Abschnitts. Ausgehend vom Zentrum des Durchgangslochs folgt die Stufe in peripherer Richtung auf den zentralen Abschnitt. Die Steigung bezeichnet den Winkel, den die Lippenoberfläche bzw. Lippenunterfläche mit der sich quer zur Axialrichtung des Durchgangslochs erstreckenden Ebene einschließt.

Ebenfalls vorteilhaft für den Eingriff der Schraube in die Lippe kann es sein, wenn die Lippe in Umfangsrichtung wellenförmig ausgebildet ist. Wenn die Lippenoberfläche eine in Umfangsrichtung geneigte Neigungsfläche aufweist, hat eine Bewegung, die der Wellenform in Umfangsrichtung folgt, eine Komponente nach oben in Richtung der Oberseite der Knochenplatte bzw. eine Komponente nach unten in Richtung der Unterseite der Knochenplatte. Dies hat den Vorteil, dass der erste Kontakt unter einem weniger spitzen Winkel stattfindet, wenn das Kopfgewinde der Schraube auf die Neigungsfläche trifft. Aufgrund des weniger spitzen Winkels ist das Risiko vermindert, dass das Gewinde auf der Lippenoberfläche entlang gleitet, anstatt in die Lippe einzuschneiden und diese umzuformen. Die Gewindeverbindung zwischen der Knochenschraube und der Knochenplatte wird also in genau der Position geformt, die die Knochenplatte beim ersten Eingriff des Gewindes in die Lippe hat.

Von Vorteil ist es, wenn der in die Knochenplatte eingeschraubte Knochenschraubenkopf möglichst wenig über die Knochenplatte hinausragt, weil ansonsten die Gefahr besteht, dass das umliegende Gewebe irritiert wird. Wenn die Lippe in der an die Unterseite der Knochenplatte angrenzenden (unteren) Hälfte des Durchgangslochs angeordnet ist, ist oberhalb der Lippe Raum, um den Kopf der Knochenschraube in der Knochenplatte zu versenken. Die an die Oberseite der Knochenplatte angrenzende (obere) Hälfte des Durchgangslochs wird dann von der Mantelfläche des Durchgangslochs gebildet, ist also frei von der Lippe.

Die Knochenplatte mit der Lippe in dem Durchgangsloch kann einstückig ausgebildet sein. Alternativ ist es möglich, dass ein das Durchgangsloch umgebender Bereich in Form eines Inlays in die Knochenplatte eingesetzt ist. Das Inlay mit der Lippe kann zunächst als separates Teil gebildet werden und dann mit der Knochenplatte verbunden werden. Um das Formen der Gewindeverbindung zu erleichtern, kann das Inlay aus einem weicheren Material bestehen als die Knochenplatte.

Um dem Chirurgen wahlweise die Verwendung konventioneller Knochenschrauben zu erlauben, deren Kopf nicht mit einem Gewinde versehen ist, kann die Mantelfläche des Durchgangslochs oberhalb der Lippe nach außen aufgeweitet sein. Dieser Bereich der Mantelfläche kann dann eine Gegenfläche für einen konventionellen Schraubenkopf bilden. Im Querschnitt kann die Aufweitung beispielsweise die Form eines Kreissegments haben.

Die Erfindung betrifft außerdem ein System aus einer solchen Knochenplatte und einer Knochenschraube. Die Knochenschraube hat ein Kopfgewinde, das dazu ausgelegt ist, die Lippe in dem Durchgangsloch umzuformen, um eine Gewindeverbindung zu bilden. Um eine sichere Befestigung an dem Knochen zu ermöglichen, hat die Knochenplatte in aller Regel eine Mehrzahl von Durchgangslöchern. Entsprechend kann auch das System eine Mehrzahl von Knochenschrauben umfassen.

Die beim Bilden der Gewindeverbindung stattfindende Materialumformung soll möglichst nicht mit Spanabrieb verbunden sein. Versuche haben gezeigt, dass der Spanabrieb gering gehalten wird, wenn der Schraubenkopf eine kegelförmige Mantelfläche aufweist und der Winkel, den die Mantelfläche mit der Schraubenachse einschließt, zwischen 19° und 28° liegt.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine Ansicht von oben auf eine erfindungsgemäße Knochenplatte;
- Fig. 2:: einen Schnitt durch Fig. 1 entlang der Linie I-I;
- Fig. 3:: die Ansicht aus Fig. 2 bei einer anderen Ausführungsform der Erfindung;
- Fig. 4:: ein Blick vom Zentrum des Durchgangslochs in Richtung der Mantelfläche bei noch einer weiteren Ausführungsform der Erfindung;
- Fig. 5 und 6:: die Ansicht aus Fig. 2 bei weiteren Ausführungsformen der Erfindung; und
- Fig. 7:: eine für die erfindungsgemäße Knochenplatte bestimmte Knochenschraube.

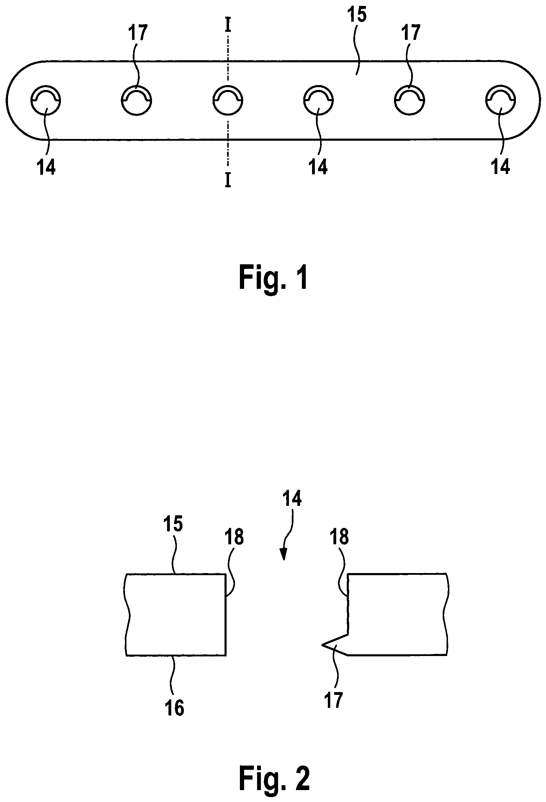

Eine erfindungsgemäße Knochenplatte hat gemäß den Figuren 1 und 2 sechs Durchgangslöcher 14, die sich von einer Oberseite 15 der Knochenplatte bis zur einer Unterseite 16 der Knochenplatte erstrecken. In jedem Durchgangsloch 14 ist über einen Teil des Umfangs eine Lippe 17 ausgebildet, die von der Mantelfläche 18 in Richtung des Zentrums des Durchgangslochs 14 vorspringt. Die Lippe 17 erstreckt sich entlang der Mantelfläche 18 über einen Umfangsabschnitt von 180°. Im verbleibenden Umfangsabschnitt der Mantelfläche von 180° gibt es keine Lippe. Die Lippe 17 schließt mit der Unterseite 16 der Knochenplatte ab, so dass oberhalb der Lippe 17 möglichst viel Raum für die Aufnahme des Schraubenkopfs bleibt.

Die Knochenplatte ist dazu bestimmt mit der Unterseite 16 auf einen Knochen aufgelegt zu werden, so dass die Knochenplatte sich über eine Bruchstelle des Knochens hinweg erstreckt. Nachdem der Knochen in die richtige Stellung reponiert ist, wird die Knochenplatte mit den Knochenfragmenten verbunden. Der Knochen ist dann in dieser Stellung fixiert und kann ausheilen.

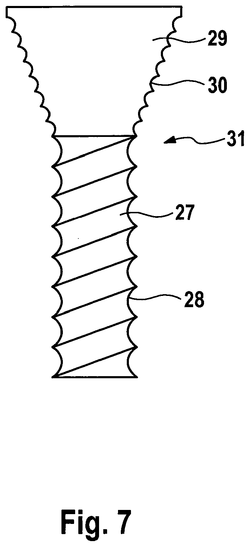

Zum Verbinden der Knochenplatte mit dem Knochen werden Knochenschrauben 31 verwendet, wie sie in Fig. 7 dargestellt sind. Die Knochenschrauben 31 haben einen Schaft 27, der mit einem Knochengewinde 28 versehen ist, und einen Schraubenkopf 29, dessen Außenfläche ein Kopfgewinde 30 mit kleinerer Steigung aufweist. Die Mantelfläche des Schraubenkopfs 29 schließt mit der Schraubenachse einen Winkel von 25° ein. Die Knochenschraube wird in den Knochen eingedreht, bis der Schraubenkopf 29 in das Durchgangsloch 14 eindringt. Dabei kann der Winkel, den die Knochenschraube mit der Achse des Durchgangslochs einschließt, zwischen etwa 0° und 15° frei gewählt werden. Wenn die Knochenschraube nun weiter eingedreht wird, kommt das Kopfgewinde 30 der Knochenschraube 31 in Eingriff mit der Lippe 17.

Dabei wird einerseits die Lippe 17 verformt, so dass sich eine Gewindeverbindung zwischen dem Kopf 29 der Knochenschraube 31 und der Lippe 17 bildet. Andererseits wird durch den zunehmenden Druck, der beim Eindrehen in die Lippe 17 entsteht, der Schraubenkopf 29 an der gegenüberliegenden Seite des Durchgangslochs 14 gegen die Mantelfläche 18 gedrückt.

Um das Bilden der Gewindeverbindung zu erleichtern, kann die Knochenschraube 31 aus einem härteren Material bestehen als die Knochenplatte. Beispielsweise kann die Knochenplatte aus reinem Titan gefertigt sein, während die Knochenschraube 31 aus der Titanlegierung TiAl6V4 besteht.

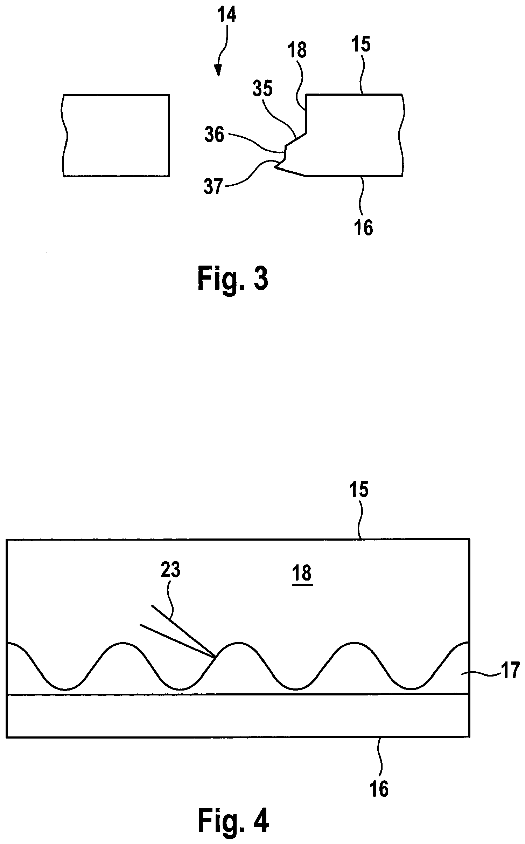

Gemäß Fig. 3 umfasst die Lippe 17 einen peripheren Bereich 35, eine Stufe 36 und einen zentralen Bereich 37. Im peripheren Bereich 35 und im zentralen Bereich 37 ist die Steigung der Lippenoberfläche klein und beträgt beispielsweise etwa 15°. Im Bereich der Stufe 36, die den zentralen Bereich 37 und den peripheren Bereich 35 verbindet, ist die Steigung wesentlich größer und beträgt beispielsweise 75°. Wenn eine Knochenschraube 31 in das Durchgangsloch 14 eingedreht wird, kommt das Kopfgewinde 30 zunächst mit dem zentralen Bereich 37 der Lippe 17 in Eingriff. Im zentralen Bereich 37 ist die Lippe dünn, so dass nur wenig Material verdrängt wird, wenn das Kopfgewinde 30 eindringt. Der Chirurg, der die Schraube eindreht, merkt kaum einen erhöhten Widerstand. Beim weiteren Eindrehen dringt das Kopfgewinde 30 weiter in den zentralen Bereich 37 ein und nähert sich an die Stufe 36 an. Wenn das Kopfgewinde 30 bei der Stufe 36 ankommt, ist das Gewinde bereits soweit ausgebildet, dass es eine sichere Führung für die Knochenschraube bietet. Wenn das Kopfgewinde 30 an der Stufe 36 anliegt, erhöht sich der Widerstand, der beim weiteren Eindrehen der Schraube überwunden werden muss, da nun eine größere Menge an Material umgeformt werden muss. Die Knochenschraube 31 wird ab dem Punkt des erhöhten Widerstands noch um 90° weiter eingedreht, so dass sich einerseits eine stabile Gewindeverbindung bildet und andererseits gegenüber der Schraubenkopf 29 mit hinreichender Kraft gegen die Mantelfläche 18 gepresst wird. Damit ist der Endzustand erreicht und das Kopfgewinde 30 ist sicher in der Lippe 17 verriegelt.

Die Fig. 4 zeigt eine Ansicht, bei der eine Draufsicht auf die Mantelfläche 18 vom Zentrum des Durchgangslochs 14 aus betrachtet in die Ebene projiziert ist. Der Bereich der Mantelfläche, der frei von der Lippe 17 ist, ist in Fig. 4 nicht sichtbar. Bei dieser Ausführungsform ist die Lippe 17 wellenförmig. Beim Eindrehen der Knochenschraube kommt zu einem gewissen Zeitpunkt der erste Gewindegang des Kopfgewindes in Kontakt mit der Lippe 17. Da der Gewindegang sich, wie in Fig. 4 bei 23 angedeutet von schräg oben an die Lippe 17 annähert, besteht eine hohe Wahrscheinlichkeit, dass der erste Kontakt zwischen dem Kopfgewinde und der Lippe 17 in einem Bereich großer Steigung stattfindet. Der Gewindegang trifft dann im Wesentlichen rechtwinklig auf die Oberfläche der Lippe 17, so dass der Gewindegang sofort in die Lippe 17 einschneiden kann und diese verformt. Es bildet sich eine Gewindeverbindung im Bereich der Lippe 17, die gleichzeitig zur Folge hat, dass der Schraubenkopf 29 auf der anderen Seite des Durchgangslochs 14 gegen die Mantelfläche gepresst wird. Dadurch wird der Schraubenkopf 29 sicher in dem Durchgangsloch 14 verriegelt.

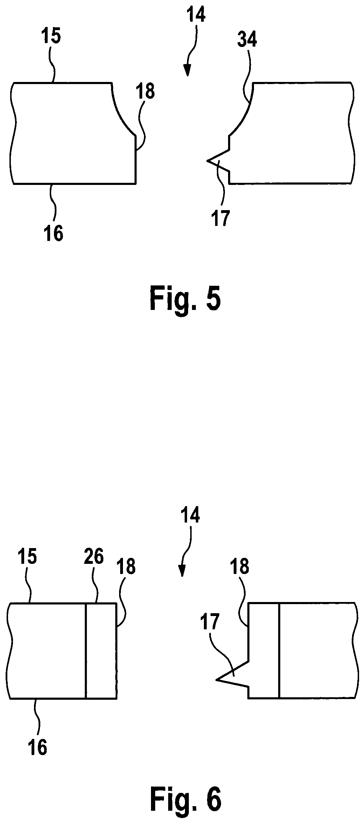

In Fig. 5 hat die Mantelfläche 18 oberhalb der Lippe 17 eine Aufweitung 34, die im Querschnitt die Form eines Kreissegments hat. Bei dieser Ausführungsform besteht wahlweise die Möglichkeit der Verwendung konventioneller Knochenschrauben, deren Kopf frei von einem Gewinde ist. Die Aufweitung 34 bildet dann eine Gegenfläche für eine Knochenschraube mit einem halbkugelförmigen Kopf, so dass die Knochenschraube unter unterschiedlichen Winkeln in das Durchgangsloch 14 eingesetzt werden kann.

Die Fig. 6 zeigt eine Ausführungsform, bei der ein Inlay 26 in die Knochenplatte eingesetzt ist. Das Inlay 26 besteht aus einem weicheren Material als die Knochenplatte und bildet die Lippe 17 sowie die Umgebung des Durchgangslochs 14.

## Patentansprüche

1. Knochenplatte mit einem Durchgangsloch (14), das sich von einer Oberseite (15) bis zu einer knochenseitigen Unterseite (16) der Knochenplatte erstreckt, wobei in dem Durchgangsloch (14) eine Lippe (17) ausgebildet ist, die von der Mantelfläche (18) des Durchgangslochs (14) vorspringt, sich in Umfangsrichtung des Durchgangslochs (14) erstreckt und dazu ausgebildet ist, vom Kopfgewinde (30) einer Knochenschraube (31) so verformt zu werden, dass sich eine Gewindeverbindung zwischen der Lippe (17) und dem Kopfgewinde (30) bildet, **dadurch gekennzeichnet, dass** die Lippe (17) sich über einen Teil des Umfangs des Durchgangslochs (14) erstreckt und dass die Mantelfläche (18) in dem der Lippe (17) gegenüberliegenden Umfangsabschnitt frei von einer Lippe ist, wobei der Umfangsabschnitt sich über mindestens 120° erstreckt.

2. Knochenplatte nach Anspruch 1, **dadurch gekennzeichnet, dass** Umfangsabschnitt, in dem die Mantelfläche (18) frei von einer Lippe ist, sich über mindestens 180°, vorzugsweise mindestens 240° erstreckt.

3. Knochenplatte nach Anspruch 1 oder 2, **dadurch gekennzeichnet, dass** die Lippe (17) eine zwischen der Mantelfläche (18) des Durchgangslochs (14) und einem zentralen Bereich (37) der Lippe (17) angeordnete Stufe (36) aufweist, die sich längs der Lippe (17) erstreckt.

4. Knochenplatte nach einem der Ansprüche 1 bis 3, **dadurch gekennzeichnet, dass** die Lippe (17) in Umfangsrichtung wellenförmig ausgebildet ist.

5. Knochenplatte nach einem der Ansprüche 1 bis 4, **dadurch gekennzeichnet, dass** die Lippe (17) in der an die Unterseite (16) der Knochenplatte angrenzenden Hälfte des Durchgangslochs (14) angeordnet ist.

6. Knochenplatte nach einem der Ansprüche 1 bis 5, **dadurch gekennzeichnet, dass** ein das Durchgangsloch (14) umgebender Bereich in Form eines Inlays (26) in die Knochenplatte eingesetzt ist.

7. System aus einer Knochenplatte nach einem der Ansprüche 1 bis 6 und einer Knochenschraube (31), wobei die Knochenschraube (31) ein Kopfgewinde (30) aufweist, das zum Umformen der Lippe (17) des Durchgangslochs (14) bestimmt ist.

8. System nach Anspruch 7, **dadurch gekennzeichnet, dass** die Knochenschraube (31) einen Kopf (29) mit einer kegelförmigen Mantelfläche aufweist und dass die Mantelfläche mit der Schraubenachse einen Winkel zwischen 19° und 28° einschließt.

## Claims

1. Bone plate with a through-hole (14) which extends from an upper side (15) of the bone plate to a lower side (16) thereof facing the bone, wherein a lip (17) is formed in the through-hole (14), which lip (17) projects from the lateral surface (18) of the through-hole (14), extends in the circumferential direction of the through-hole (14) and is designed to be deformed by the head thread (30) of a bone screw (31) such that a threaded connection forms between the lip (17) and the head thread (30), **characterized in that** the lip (17) extends over a part of the circumference of the through-hole (14), and **in that** the lateral surface (18) is free of a lip in the circumferential portion lying opposite the lip (17), wherein the circumferential portion extends over at least 120°.

2. Bone plate according to Claim 1, **characterized in that** the circumferential portion in which the lateral surface (18) is free of a lip extends over at least 180°, preferably at least 240°.

3. Bone plate according to Claim 1 or 2, **characterized in that** the lip (17) has a step (36) arranged between the lateral surface (18) of the through-hole (14) and a central region (37) of the lip (17), said step (36) extending along the lip (17) .

4. Bone plate according to one of Claims 1 to 3, **characterized in that** the lip (17) is configured with an undulating shape in the circumferential direction.

5. Bone plate according to one of Claims 1 to 4, **characterized in that** the lip (17) is arranged **in that** half of the through-hole (14) that is adjacent to the underside (16) of the bone plate.

6. Bone plate according to one of Claims 1 to 5, **characterized in that** a region surrounding the through-hole (14) and in the form of an inlay (26) is inserted into the bone plate.

7. System composed of a bone plate according to one of Claims 1 to 6 and of a bone screw (31), wherein the bone screw (31) has a head thread (30) which is intended to deform the lip (17) of the through-hole (14).

8. System according to Claim 7, **characterized in that** the bone screw (31) has a head (29) with a conical lateral surface, and **in that** the lateral surface encloses an angle of between 19° and 28° with the screw axis.

## Revendications

1. Plaque d'ostéosynthèse comprenant un trou de passage (14) qui s'étend depuis un côté supérieur (15) jusqu'à un côté inférieur (16) côté os de la plaque d'ostéosynthèse, une lèvre (17) étant réalisée dans le trou de passage (14), laquelle fait saillie depuis la surface d'enveloppe (18) du trou de passage (14), s'étend dans la direction périphérique du trou de passage (14) et est réalisée de manière à être déformée par le filetage de tête (30) d'une vis d'ostéosynthèse (31) de telle sorte qu'une liaison par filetage entre la lèvre (17) et le filetage de tête (30) soit formée, **caractérisée en ce que** la lèvre (17) s'étend sur une partie de la périphérie du trou de passage (14) et **en ce que** la surface d'enveloppe (18) dans la portion périphérique opposée à la lèvre (17) ne présente pas de lèvre, la portion périphérique s'étendant sur au moins 120°.

2. Plaque d'ostéosynthèse selon la revendication 1, **caractérisée en ce que** la portion périphérique dans laquelle la surface d'enveloppe (18) ne présente pas de lèvre s'étend sur au moins 180°, de préférence sur au moins 240°.

3. Plaque d'ostéosynthèse selon la revendication 1 ou 2, **caractérisée en ce que** la lèvre (17) présente un étage (36) disposé entre la surface d'enveloppe (18) du trou de passage (14) et une région centrale (37) de la lèvre (17), qui s'étend le long de la lèvre (17).

4. Plaque d'ostéosynthèse selon l'une quelconque des revendications 1 à 3, **caractérisée en ce que** la lèvre (17) est réalisée sous forme ondulée dans la direction périphérique.

5. Plaque d'ostéosynthèse selon l'une quelconque des revendications 1 à 4, **caractérisée en ce que** la lèvre (17) est disposée dans la moitié du trou de passage (14) adjacente au côté inférieur (16) de la plaque d'ostéosynthèse.

6. Plaque d'ostéosynthèse selon l'une quelconque des revendications 1 à 5, **caractérisée en ce qu'**une région entourant le trou de passage (14) en forme d'insert (26) est insérée dans la plaque d'ostéosynthèse.

7. Système constitué d'une plaque d'ostéosynthèse selon l'une quelconque des revendications 1 à 6 et d'une vis à os (31), dans lequel la vis à os (31) présente un filetage de tête (30) qui est prévu pour déformer la lèvre (17) du trou de passage (14) .

8. Système selon la revendication 7, **caractérisé en ce que** la vis à os (31) présente une tête (29) avec une surface d'enveloppe de forme conique et **en ce que** la surface d'enveloppe forme avec l'axe de vis un angle compris entre 19° et 28°.
